Europäisches Patentamt

European Patent Office

Office européen des brevets

(11) Publication number: **0 241 907**
A2

# (12) EUROPEAN PATENT APPLICATION

(21) Application number: **87105504.2**

(22) Date of filing: **14.04.87**

(51) Int. Cl.4: **A61K 39/395** , A61K 49/00 ,
C07K 15/00 , C12P 21/00 ,
//C12N5/00,C12N15/00,(C12P2-
1/00,C12R1:91)

The applicant has filed a statement in accordance with Rule 28 (4) EPC (issue of a sample only to an expert). Accession number(s) of the deposit(s): ATCC HB-9090.

(30) Priority: **14.04.86 US 851554**

(43) Date of publication of application:
**21.10.87 Bulletin 87/43**

(84) Designated Contracting States:
**AT BE CH DE ES FR GB GR IT LI LU NL SE**

(71) Applicant: **THE GENERAL HOSPITAL CORPORATION**
**55 Fruit Street**
**Boston MA 02114(US)**

(72) Inventor: **Haber, Edgar**
**83, Ridgeway Road**
**Weston Massachusetts 02193(US)**

(74) Representative: **Vossius & Partner**
**Siebertstrasse 4 P.O. Box 86 07 67**
**D-8000 München 86(DE)**

(54) **Heterobifunctional antibodies and method of use.**

(57) This invention relates to heterobifunctional antibodies having dual specificities, one specificity directed against a thrombus, and the other specificity directed against a thrombolytic agent. The invention also relates to methods of using these heterobifunctional antibodies.

EP 0 241 907 A2

## HETEROBIFUNCTIONAL ANTIBODIES AND METHOD OF USE

This invention relates to heterobifunctional antibodies having dual specificities, one specificity directed against a thrombus and the other specificity directed against a thrombolytic agent. This invention further relates to a method of using these heterobifunctional antibodies in immunodiagnostic and immunotherapeutic processes.

Most myocardial infarctions are caused by coronary thrombosis (DeWood et al., N. Eng. J. Med., 303: 897 (1983). The coronary thrombosis that causes the myocardial infarction can be lysed by thrombolytic agents. These thrombolytic agents are plasminogen activators that activate the conversion of plasminogen to the fibrinolytic enzyme plasmin. Plasmin will then lyse the fibrin present in the thrombus. This treatment with plasminogen activators is not without side effects. Plasmin acts nonselectively and therefore, not only lyses the fibrin in the thrombus, but also attacks fibrinogen and clotting factors, often resulting in severe bleeding diathesis.

Streptokinase, urokinase and tissue-type plasminogen activator (TPA) are three known plasminogen activators for lysing thrombi. These activators are indicated for the treatment for acute cardiovascular disease such as infarct, stroke, pulmonary embolism, deep vein thrombosis, peripheral arterial occlusion, and other venous thrombosis. Both streptokinase and urokinase, however, have severe limitations. Due to a low affinity for fibrin, both activators will activate circulating and fibrin-bound plasminogen indiscriminately. The plasmin formed in circulating blood is neutralized before it can be used in thrombolysis. Residual plasmin will degrade several clotting factor proteins, for example, fibrinogen, factor V, and factor VIII, causing hemorrhagic potential. Further, streptokinase is strongly antigenic and patients with high antibody titers respond inefficiently to treatment and cannot remain on continuous treatment.

Human tissue-type plasminogen activator can bind to fibrin and therefore favors the activation of plasminogen in close proximity to the thrombus, potentially sparing fibrinogen elsewhere in the circulation. However, at doses required for prompt lysis of coronary thrombi, the use of tissue-type plasminogen activator can also result in hemorrhage.

In order to increase the specificity of the thrombolytic agents to the thrombus, it has been shown that covalent linkage of urokinase to a fibrin-specific antibody results in marked enhancement of fibrinolytic potency and specificity Bode et al., Science, 229:765-767 (1985).

One function characteristic of every antibody molecule is specific binding to an antigenic determinant. Antibodies in vivo are bivalent and monospecific, containing two identical antigen binding sites. The specific binding of antigen by an antibody molecule is determined by the antibody's structure of the variable regions ($F_{ab}$) of both heavy and light chains. Antibodies having dual specificities have been prepared by subjecting antibodies of different specificities to a selective cleavage of the disulfide bridges that link the two heavy chains together. Antibody half-molecules are then reassociated under neutral pH to produce the hybrid antibodies having dual specificities.

Nisonhoff et al., Nature (London), 194: 355 (1962) describes the in vitro production of a bispecific antibody molecule from a polyclonal rabbit antibody, anti-ovalbumin, and an anti-bgg antibody. The monospecific antibodies were treated with pepsin to remove the $F_c$ portion of the antibody, leaving the two antigen-binding sites ($F_{ab}$) covalently linked by a single disulfide bond. This bond was then split under reducing conditions and the two antibody molecules reassociated under oxidizing conditions to produce a bispecific antibody.

In Brennan et al., Science, 229: 31 (1985) a chemical procedure is described for preparing bispecific antibody fragments from monoclonal antibodies. In this procedure, a modification of the Nisonoff technique was used in cleaving the $F_{ab}$ fragments, followed by reconstituting the half-fragments to form the bispecific antibody molecule. The $F_{ab}$ fragments were reduced in the presence of sodium arsenite to stabilize vicinal dithiols and impede intramolecular disulfide formation. The other modification involved activating the thiols of one of the half-$F_{ab}$ fragments as a thionitrobenzoate derivative. By this process, a bispecific antibody was produced from anti-avidin $F_{ab}$ and anti-luciferase $F_{ab}$ was produced.

Liu et al., Proc. Nat'l. Acad. Sci. USA, 82: 8648 (1985) discloses a chemical procedure for forming a bispecific antibody in which anti-T3 antibody was covalently linked to a second monoclonal antibody, anti-Igld specific for the idiotype of the surface immunoglobulin of a human B lymphoma. The anti-T3 and anti-Igld antibodies were first reacted with N-succinimidyl-3-(2-pyridyldithio) propionate (SPDP). Thiol groups were attached to the cleaved anti-T3 antibody using 2-iminothiolane. Then the two modified half-antibodies, anti-T3 and anti-Igld, were mixed to covalently link the two antibodies.

The result showed that the T8 cytotoxic T lymphocytes lysed the human B-lymphoma cells, but no lyses was observed when T4 cytotoxic T lymphocyte cells were used.

Bispecific antibodies have also been produced from hybridomas. The preparation of bispecific monoclonal antibodies by fusion of antibody-producing hybridoma cells is described in Milstein and Cuello, Nature (London), 305: 537 (1983). This reference describes the fusion of two hybridomas, or the fusion of one hybridoma with spleen cells from an immunized rat, to produce hybrid hybridomas. These hybrid hybridomas secrete predefined bispecific monoclonal antibodies as well as monospecific antibodies. Anti-somatostatin/anti-pluroxidase and anti-substance P/ anti-peroxidase bispecific monoclonal antibodies were prepared in this manner. The bispecific monoclonal antibodies produced by hybrid hybridomas were complete molecules, containing the $F_c$ region as well as the antigen-combining sites.

PCT application, WO83/03679, describes the production of a bispecific antibody having dual specificities obtained by fusion two hybridomas. This application describes procedures for producing and selecting hybrid hybridomas. The bispecific antibodies therein are described as having many potential uses, ranging from immunodiagnostic procedures to targeted delivery of drugs.

It would be desirable to have a bispecific antibody having dual specificity such that one specificity would be directed against a thrombus and the other specificity would be directed against a thrombolytic agent. With this bispecific antibody, a thrombus would be detected. This thrombus then could be lysed by the action of a thrombolytic agent that becomes or is attached to the anti-thrombolytic antibody. The lysis of thrombi is complicated; it was not known whether a bispecific antibody would block or inhibit the thrombolytic activity by the thrombolytic agent.

The present invention provides for a heterobifunctional antibody having dual specificity, with one specificity directed against a thrombus and the other specificity directed against a thrombolytic agent. The heterobifunctional antibody according to this invention can be used for immunodiagnosis and immunotherapy. Accordingly, the present invention also provides processes for immunodiagnosis and immunotherapy employing the heterobifunctional antibodies.

Figure I shows the release of labeled fibrin from fibrin-Sepharose by the following compounds: (I) a heterobifunctional antibody specific for both fibrin and tissue plasminogen activator (TPA) complexed to TPA (BI-AB), then TPA); (2) a heterobifunctional antibody specific for both fibrin and TPA, first added to fibrin-Sepharose followed by the addition of TPA after washing (BI-AB + TPA); (3) a urokinase-antifibrin antibody covalent complex (UK-AB); (4) TPA alone; and (5) urokinase alone (UK). Lysis was expressed as the quotient of released radioactivity and total radioactivity. Each point represents the mean of three separate experiments with a mean standard deviation of I.23.

Figure 2 shows the release of labeled peptides from fibrin-Sepharose (I) by TPA alone and (2) by TPA captured by a heterobifunctional antibody specific for both fibrin and TPA. For clarity, data at 0.I and 0.34 ng/ml are not shown in this figure but are incorporated into Figure 3. Each point represents the mean of three experiments with a mean standard deviation of 0.74.

Figure 3 shows the enhancement of fibrinolysis computed as the maximal quotient of percent lysis in the presence and absence of the heterobifunctional antibody. The data are taken from those shown in Figure 2, with the addition of experiments at 0.I and 0.3 ng/ml, not shown in Figure 2. Error bars represent standard deviations of the mean of the quotients. This figure shows that the relative potency of TPA in the antibody-pretreated samples increases as TPA concentration decreases.

Figure 4 shows the demonstration of antigen binding by heterobifunctional antibodies F32.I and F36.23 produced by somatic fusion.

Figure 5 shows the ability of the heterobifunctional antibodies F32.I and F36.23 to enhance fibrinolysis.

This invention is directed to heterobifunctional antibodies having dual specificity with one specificity directed against a thrombus and the other specificity directed against a thrombolytic agent. The individual specificities are to (a) antigenic determinants on a thrombus and (b) antigenic determinants on a thrombolytic agent.

Throughout this specification, the term "heterobifunctional antibody" is used to designate a single antibody molecule having two specificities or two molecules linked to each other each having different specificities. Other terms have been used to describe heterobifunctional antibodies including heteroantibody, bispecific antibody, hybrid antibodies, heteroligating antibody, antibody duplex, hetereodimer, among others. It will be understood by one of skill in the art that these are all equivalent terms.

The antibodies usable in preparing the heterobifunctional antibodies of the present invention may be either polyclonal or monoclonal antibodies. In the preferred embodiments of this invention, monoclonal antibodies are used in preparing heterobifunctional antibodies.

The anti-thrombus specificity as used herein refers to antibodies raised against fibrin or fibrinogen. Blood clots are formed when thrombin cleaves two pairs of small peptides from fibrinogen to yield fibrin monomers (Blomback et al., Ark. Kemi., 12: 173 (1958) and Doolittle, R.F., Adv. Protein Chem., 27: 1 (1973)). Fibrin monomers spontaneously aggregate to form an insoluble gel that is covalently stabilized by Factor XIIIa. Fibrin retains 98% of the original covalent structure of fibrinogen. Thus, in the preferred embodiment of this invention the antibodies that are used to form one-half of the heterobifunctional molecule are any antibodies which are fibrin-specific and are substantially devoid of fibrinogen cross-reactivity.

For example, antibodies with this specificity have been described in Hui et al., Science, 222: 1129 (1983). Further description of the same type of antibodies can be found in Abstract 1375, 74th Annual Meeting of the American Society of Biological Chemists, by Gary R. Matsueda et al. for "Fibrin-Specific Monoclonal Antibodies Lacking Fibrinogen Cross-Reactivity." Fibrinspecific monoclonal antibodies with substantially no fibrinogen cross-reactivity are also described in European Application No.      filed concurrently herewith. Other examples of antibodies with a specificity against a thrombus include Kudryk et al, Mol. Imm., 21: 89 (1984). All of the above references are herein incorporated by reference.

Antibodies specific against thrombolytic agents may also be polyclonal or monoclonal antibodies, preferably monoclonal antibodies. When plasminogen is converted by an activator to plasmin, the active fibrinolytic enzyme of plasma, it develops a marked affinity for its substrate, fibrin. Three plasminogen activators are currently available for converting plasminogen to plasmin: streptokinase, urokinase, and human tissue plasminogen activator (TPA). The term "thrombolytic agent" as used in this specification is therefore meant to include broadly any agent utilized for inducing or initiating the lysis of a thrombus. Other terms are known in the art for the lysis of a thrombus, including fibrinolysis. Although the most common thrombolytic agents are streptokinase, urokinase, and tissue-type plasminogen activator, any other thrombolytic agent can be utilized as defining the specificity of the relevant portion of the heterobifunctional antibodies of the invention.

Antibodies specific against thrombolytic agents may be raised according to means known in the art. In order to have enhanced specificity, it is preferred that monoclonal antibodies be raised against the thrombolytic agents. (Kohler and Milstein, Nature, 256:495 (1975).

The process for obtaining a heterobifunctional antibody according to the present invention requires one antibody specific against a thrombus and the other antibody specific against a thrombolytic agent. These two antibodies may be modified by chemical procedures for preparing antibody fragments therefrom that can then be recombined to produce the heterobifunctional antibody. Alternatively, the heterobifunctional antibody can be prepared from the fusion of two hybridomas producing a hybrid hybridoma which secretes predefined heterobifunctional antibodies.

One procedure in which the anti-thrombus antibody and the anti-thrombolytic agent antibody may be chemically modified to produce the heterobifunctional antibody is described in Liu et al, Proc. Nat'l. Acad. Sci. (U.S.A.), 82: 8648 (1985), incorporated herein by reference. In this procedure, each antibody is treated separately with a molar excess of N-succinimidyl-3(2-pridyldithio) propionate (SPDP) dissolved in absolute ethanol. Thiol groups are then added to one of the modified antibodies by reacting a molar excess of 2-iminothiolanine dissolved in sodium borate. Then equal molar amounts of the two modified antibodies are mixed and after sufficient reaction time, the reaction is stopped by adding an excess of iodoacetamide. The reaction mixture is then passed over an affinity column to separate the bispecific antibody from uncoupled antibodies. In this procedure, the $F_c/F_{ab}$ one-half of the antibody molecule is kept intact.

In another chemical procedure for producing heterobifunctional antibodies, the $F_c$ fraction of the antibody is cleaved from the $F_{ab}$ fraction. This procedure is described in Brennan et al., Science, 229: 31 (1985), incorporated herein by reference. In this procedure, the $F_c$ portion of the antibody is cleaved by pepsin hydrolysis to yield the $F_{ab}$ portion. The $F_{ab}$ portion is then reduced by mercaptoethylamine in the presence of sodium arsenite to cleave the disulfide bonds. The bonds are stabilized with Ellman's reagent, 5,5′-dithiobis(2-nitrobenzoic) acid. One of the stable $F_{ab}$ half-molecules is then treated with mercaptoethylamine and is mixed with an equimolar amount of the other untreated stable $F_{ab}$ half-molecule to form the heterobifunctional antibody.

As usual herein, the term "modification" refers to monospecific antibodies that are chemically altered, such as in the above two described procedures, to disassociate the antibodies and then reassociate them, producing the heterobifunctional antibody having dual specificity.

Alternatively, the heterobifunctional antibody may be produced by the fusion of hybridomas to produce a hybrid hybridoma that secretes predefined bispecific heterobifunctional antibodies. This procedure is described in Milstein and Cuello, Nature (London), 305: 537 (1983), incorporated herein by reference. Methods for producing a hybrid hybridoma which secretes a hybrid monoclonal antibody having dual specificity against two different antigenic determinants is also described in PCT application, WO 83/03679, also incorporated herein by reference. In the PCT application a procedure is described for using hybridomas with selectable markers, such that the monospecific hybridoma cannot survive in a medium in which the hybrid hybridoma is cultured. Thus, by the fusion of the two hybridomas, each conferring to the other the ability to grow in selected medium, the hybrid hybridoma can be easily selected. Examples of such selectivity include the inability to produce the enzyme HPRT, HAT-ouaban selection, HAT sensitivity, and antibiotic resistance.

The present invention also provides methods for immunotherapy and immunodiagnosis using heterobifunctional antibodies having a dual specificity, wherein one of the dual specificities is against a thrombus and the other specificity is against a thrombolytic agent. In the immunotherapeutic and immunodiagnostic applications, heterobifunctional antibodies produced from either chemical means or hybrid hybridomas can be used.

The heterobifunctional antibody of dual specificity can be used in immunotherapy by constructing it to have a specificity against a thrombus and a specificity against a thrombolytic agent. In the preferred embodiment of this invention, the anti-thrombus antibody is a monoclonal antibody against fibrin with substantially no cross-reactivity to fibrinogen and the thrombolytic agent is TPA. In this application, after the heterobifunctional antibody is administered to the patient, the heterobifunctional antibody then becomes localized at the site of the thrombus. During this time, the endogenous TPA will become attached to the heterobifunctional antibody.

Surprisingly, it has been found by the inventors that endogenous TPA is captured by the heterobifunctional antibody, enhancing the potency of endogenous TPA and fibrinolysis with increasing efficacy at decreasing concentrations of TPA (See example below).

This method may also be used by first administering to the patient a low dosage of a thrombolytic agent. The heterobifunctional antibody is then administered to the patient, and it localizes at the site of the thrombus. The heterobifunctional antibody will capture the administered thrombolytic agent, directly it to the thrombus site. In results shown in in vitro testing, the hetereobifunctional antibody may be first administered, followed by administration of a low dosage of the thrombolytic agent. As will be appreciated by one of skill in the art, the low dosage of the thrombolytic agent will reduce the risk of serious side effects, such as hemorrhage.

In another embodiment of this invention, the thrombolytic agent, for example streptokinase, uroki nase, or TPA, is attached to the heterobifunctional antibody prior to administration to the patient. In this drug-targeted system, the specificity of the anti-thrombus portion of the heterobifunctional antibody permits selectivity of the antithrombolytic agent to lyse the thrombus.

The heterobifunctional antibody may also be used in immunodiagnostic applications. In vivo immunodiagnosis can be performed using the heterobifunctional antibody of this invention. The heterobifunctional antibody, having one specificity against a thrombus and the second specificity against a thrombolytic agent, is first administered to the patient. After sufficient time has passed for the antibody to localize at the thrombus and unbound antibody has been permitted to clear from healthy tissue in the patient, the thrombolytic agent bearing a radionuclide is administered. The radionuclide must be of the type of decay which is detectable for a given type of instrument. Further, the radionuclide for in vivo diagnosis should have a half-life long enough that it is still detectable at the time of maximum uptake, but short enough that after diagnosis unwanted radiation does not remain in the patient. Coupling of the radionuclides to the protein agents is known in the art and is often accomplished either directly or indirectly using an intermediary functional group. Examples of radioisotopes that can be used for in vivo diagnosis are $^{99}$Tc, $^{123}$I, $^{131}$I, $^{111}$In, $^{97}$Ru, $^{67}$Cu, $^{67}$Ga, $^{68}$Ga, $^{72}$As, $^{89}$Zr, and $^{201}$Tl.

Paramagnetic isotopes for purposes of in vivo diagnosis can also be used according to the methods of this invention. Examples of elements that are par ticularly useful for use in Magnetic Resonance Energy techniques include $^{157}$Gd, $^{55}$Mn, $^{162}$Dy, $^{52}$Cr, and $^{56}$Fe.

The heterobifunctional antibody having dual specificity can further comprise a pharmaceutical composition, with a pharmaceutically acceptable carrier. These carriers are well known in the art and can include aqueous or solvent emulsions or suspensions, including saline and buffered media. The pharmaceutical compositions may be prepared by any of the methods that are well-known in the pharmaceutical art, for example, as described in Remington's Pharmaceutical Sciences (16th Edition, 1980).

The dose ranges for administration of the heterobifunctional antibody are those that are large enough to detect the presence of thrombi. The dosage should not be so large as to cause adverse side effects, such as unwanted cross rashes, and anaphylactic rashes and the like. Generally, the dosage will vary with the age, condition, sex, and extent of disease in the patient. Counter indications can include immune tolerance and other variables and can be adjusted by the individual physician. Dosage can range from 0.01 mg/kg to 500 mg/kg of body weight, preferably 0.01 mg/kg to 200 mg/kg. The heterobifunctional antibodies can be administered parentally by injection or by gradual perfusion over time. They can also be administered intravenously, intraperitoneally, intramuscularly, or subcutaneously.

Having now generally described this invention, the same will become more readily understood by reference to specific examples included herein for purposes of illustration only, and are not intended to be limiting unless otherwise specified.


EXAMPLES

Example I

PREPARATION OF MONOCLONAL ANTIBODIES SPECIFIC FOR HUMAN TISSUE TYPE PLASMINOGEN ACTIVATOR (ANTI-tPA)

IgGI mouse monoclonal antibodies specific for human tissue-type plasminogen activator (anti-TPA) were raised from A/J mice that had been immunized with human recombinant two-chain tissue-type plasminogen activator (TPA) (Genentech Inc). To select the appropriate clones, spleen cells from the immunized mice were fused with SP2/0 myeloma cells and the mixture was then distributed into ten 96-well microtiter plates containing macrophage feeder layers. Hybridoma colonies were observed in over 95% of the wells. Supernatants from 12 wells showed evidence of anti-TPA binding in a solid phase immunoassay. The cloning and subcloning of these colonies by the limiting dilution method resulted in 33 stable lines, each producing anti-TPA antibody. Of these stable lines, 27 were IgG, Kappa and 6 were IgG$_{2b}$ Kappa as isotyped by the Boehringer kit. Two IgG$_1$ lines were selected for expansion in ascites and the antibodies subsequently purified by ammonium sulfate precipitation (45%) and DEAE-cellulose chromatography. The resulting antibody preparations were homogeneous by SDS-PAGE electrophoresis and demonstrated binding to the heavy chain of TPA by Western blotting. The apparent dissociation constants of the two anti-TPA antibodies was estimated by determining the half maximal binding concentration of $^{125}$I-TPA. One antibody was determined to have a K$_d$ of 4.5 $\times$ 10$^{-10}$ and the other 6.5 $\times$ 10$^{-10}$. Neither antibody

inhibited the activity of TPA as measured either against an esterase substrate, S2444, or in a fibrinolysis assay against fibrin monomer (described in Example 3, below). The anti-TPA hybridoma cell line TCL8 was placed on deposit at the American Type Culture Collection (ATCC), Rockville, Maryland and designated ATCC No. HB9090. Hybridoma line TCL8 produces a monoclonal antibody specific for the catalytically active chain of TPA.


Example 2

PREPARATION OF HETEROBIFUNCTIONAL ANTIBODY HAVING DUAL SPECIFICITY FROM FIBRIN SPECIFIC MONOCLONAL ANTIBODY AND ANTI-TPA MONOCLONAL ANTIBODY

An IgGI mouse monoclonal antibody specific for fibrin and not cross-reacting with fibrinogen (59D8) has been previously described in Hui, et al., Science, 222:1129-1131 (1983). Anti-TPA monoclonal antibody described in Example I was covalently coupled to anti-fibrin monoclonal antibody 59D8 utilizing the cross-linking reagent (N-succinimidyl 3-(2 pyridyldithio) propionate (SPDP). Lui et al., Proc Nat'l. Acad. Sci. USA, 82:8648-8652 (1985). In a typical experiment, 8.4 mg anti-fibrin antibody at a concentration of 2.4 mg/ml in 0.01M phosphate, 0.15M NaCl, 0.02% NaN$_3$, pH 7.4, (PBSA) was reacted with 50 ul of 20mM SPDP in absolute ethanol. After 30 minutes at room temperature, the reagents were removed by gel filtration on a Sephadex G-25 column (2.5 $\times$ 30 cm) equilibrated with 0.14M NaCl, ImM KCl, 3.7mM sodium phosphate, pH 7.4 (NaPi). A modification of from two to four 2-pyridyl disulfide groups per more of anti-fibrin antibody was obtained. Grassetti et al., Arch. Biochem. Biophys. , 119:41-49 (1967); Stuchbury et al., Biochem. J., 151:417-432 (1975).

Thiol groups were attached to the anti-TPA antibodies by reacting 8.4 mg of either of the two anti-TPA monoclonal antibodies at a concentration of 2.4 ng/ml in NaPi with a 200-fold molar excess of 2-iminothiolane in 25mM sodium borate, pH 9.1. After 45 minutes at room temperature, the mixture was fractionated on a Sephadex G-25 column (2.5 $\times$ 30 cm) equilibrated with 0.1M NaCl, 0.1M sodium phosphate, pH 6.6. This protocol introduces I to 2 thiol groups per antibody molecule. Lui et al., supra.

Equimolar amounts of the anti-fibrin and anti-TPA modified antibodies were mixed and stirred at room temperature for 3.5 hours. The reaction was then stopped by addition of 0.5 ml IM iodoacetamide in IM sodium phosphate, pH 8.0. At this point the mixture was no longer reactive with Ellman's reagent.

The sample was next concentrated to a volume of 9 ml in a I0 ml Amicon ultrafiltration cell using a YM 30 membrane and then applied to a calibrated Sephacryl S-300 column (2.5 × 85 cm) equilibrated with 0.IM phosphate, 0.1 M NaCl, I.0M urea, pH 6.6. Two peaks were clearly resolved. The first peak, eluting at approximately 300 Kd, was consistent with heterobifunctional antibodies (heterodimers). The second peak, eluting at I50 Kd, was presumed to contain unreacted antibody monomers. Small amounts of higher molecular weight materials were also observed, which were presumed to be higher polymers. The material from the 300 Kd peak was pooled and dialyzed against the buffer to be used in subsequent assays.

To bind TPA to the heterobifunctional antibody, 3.5 mg of TPA (0.5 mg/ml) was mixed with 5 mg of heterobifunctional antibody (0.5 mg/ml) for 2 hours at room temperature. The solution was concentrated to a volume of 9 ml in a I0 ml Amicon ultrafiltration cell on a YM 30 membrane and applied to a Sephacryl S-300 column. Two peaks were resolved. One peak eluted with a slightly smaller volume than that of the heterobifunctional antibody peak, and with a molecular weight of approximately 400 Kd. Since the apparent molecular weight of TPA was 70 Kd on this column, the first peak was presumed to be an antibody-TPA complex and the second peak unbound TPA. On the basis of enzymatic activity (described in Example 3), it was estimated that about I.5 moles of TPA were bound to each mole of heterobifunctional antibody.

## EXAMPLE 3

## PEPTIDASE ENZYMATIC ACTIVITY ASSAY

The plasminogen-activating potency of TPA, the heterobifunctional fibrin-TPA antibody, urokinase, and a covalent complex of urokinase and a fibrin-specific antibody (urokinase-antibody conjugate) (Bode et al., Science, 229:765-767 (I985)), were compared at equivalent peptidase activities. Peptidase activity independent of fibrin binding was measured with the chromogenic substrate, S-2288 (Helena Labs). Urokinase (Abbokinase, Abbot Lot #82-087-AF)) was the reference standard. The peptidase activity of I unit of urokinase was equal to that of 7 ng of TPA.

## EXAMPLE 4

## PLASMINOGEN ACTIVATOR ASSAY

The lysis of $^{125}$I-fibrin monomer covalently linked to Sepharose 4B-Cl (fibrin-Sepharose) provided the end-point for the plasminogen-activator assay. Hui et al., supra. In this assay, the test substance was incubated with a 300 ul of a suspension of fibrin-Sepharose in a buffer containing I0 mM sodium phosphate, 0.1% BSA, 0.01% Tween-80, pH 7.4. The fibrin-Sepharose was washed with the same buffer and then incubated with I ml of plasminogen solution (0.I5 mg/ml). Release of labeled peptides was measured at varying intervals by counting aliquots of the supernatant after centrifugation of the fibrin-Sepharose and was expressed as a percentage of the total initial radioactivity.

The test substances assayed included:

    i) the heterobifunctional antibody described in Example 2;

    ii) the heterobifunctional antibody pretreated with TPA;

    iii) TPA

    iv) urokinase (UK), and

    v) urokinase-anti-fibrin conjugate.

An additional sample, consisting of the heterobifunctional antibody that had been incubated with fibrin-Sepharose, washed, mixed with TPA, and washed again, was incubated with plasminogen as described above.

Figure I shows that the concentration of TPA required to release labeled peptides from fibrin-Sepharose is about I/I0 that of urokinase and that the TPA-heterobifunctional antibody is I0-fold more potent than TPA alone. There was no significant difference in fibrinolytic effectiveness between a heterobifunctional antibody that had been treated first with TPA and then added to the fibrin-Sepharose, and that of heterobifunctional antibody mixed first with fibrin-Sepharose and then treated with TPA. The TPA-heterobifunctional antibody is equipotent to the urokinase-anti-fibrin complex described in Bode et al., supra.

The relative efficacy of TPA relative to urokinase has been attributed to the capacity of TPA to selectively bind to fibrin. Collen et al., Circulation, 70:I0I2-I0I7 (I984); Bergmann et al ., Science, 220:II8I-II83 (I983); Hoylaerts et al., J. Biol. Chem., 257:29I2-29I9 (I982). Urokinase does not have a fibrin binding site. The even greater fibrinolytic potency observed with the TPA-heterobifunctional antibody, and with the urokinase-anti-fibrin complex, may be explained by the considerable difference in the relative affinity for fibrin that exists between TPA and fibrin-specific antibody 59D8. The $K_d$ of TPA for fibrin is 0.I mM,

whereas that of antibody 59D8 is approximately 0.1 microM. In the heterobifunctional antibody, the affinity of anti-TPA for TPA is 0.1nM, not a limiting factor.

## EXAMPLE 5

### TPA CAPTURE AT LOW CONCENTRATIONS

The plasma concentration of TPA is reported to be in the range of 5ng/ml. Hamster et al., N. Engl. J. Med., 313:1557-63 (1985). To determine whether the fibrinolytic efficacy of TPA would be enhanced at these and lower TPA concentrations, 1 ml aliquots of fibrin-Sepharose were incubated both with or without 330 ug/ml heterobifunctional antibody in a volume of 1.0 ml for 4 hours at 20°C. A volume of 2.7 1 of 100 mM phosphate, 0.1% BSA, 0.01% Tween-80 containing 0 (control), 0.1, 0.3, 1.0 or 5.0 ng/ml TPA was then run through a column at a flow rate of 180 ml/hr. After the fluid had passed through the column, the fibrin-Sepharose was removed and placed in a test tube at 25°C. One ml of plasminogen (0.15 1mg/ml) was then added. The Sepharose was allowed to settle at the bottom of the test tube. At 20, 45, 60, 90, 120, 150 and 180 min, 0.6 ml of the supernatant solution was removed, counted in a gamma scintillation counter and then returned to the fibrin-Sepharose. Percent lysis was computed as the fraction of total counts released into the supernatant.

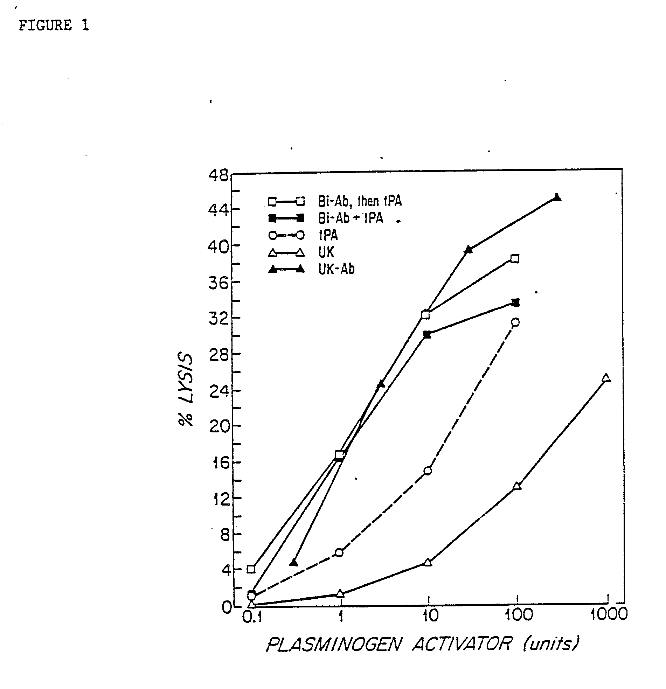

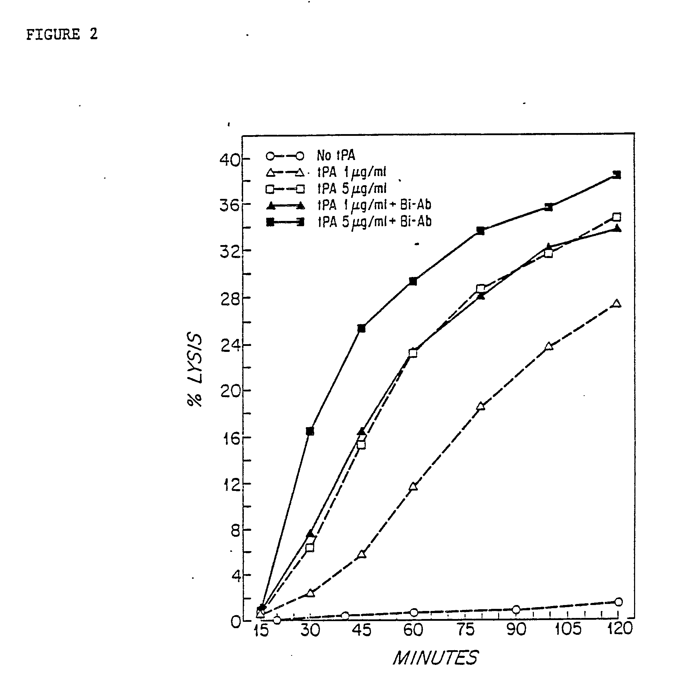

Figure 2 compares the fibrinolytic rates by TPA, both with and without pretreatment of fibrin-Sepharose with the heterobifunctional antibody. At each TPA concentration, the data indicate substantially enhanced fibrinolysis in the samples heated with heterobifunctional antibody.

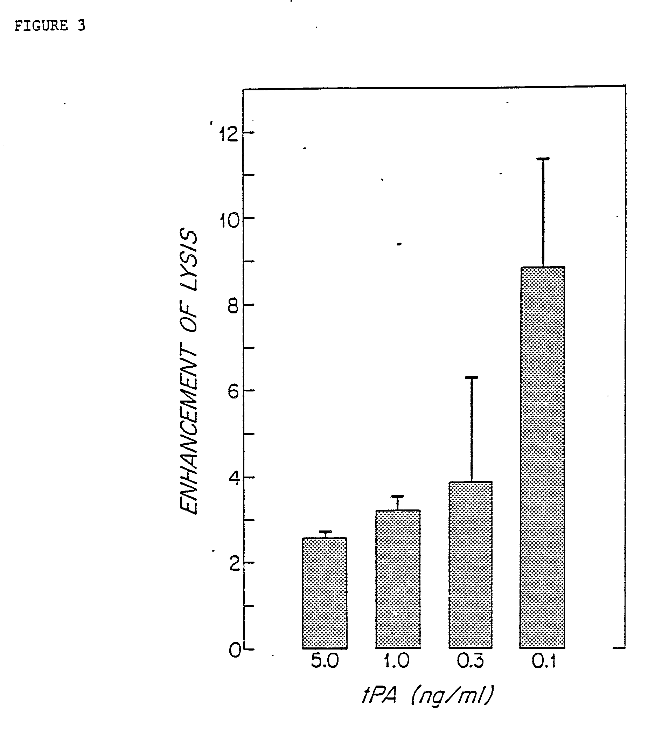

In Figure 3, enhancement of lysis is defined as the ratio maximal of fibrinolysis in the presence and absence of heterobifunctional antibody. It is apparent that the relative potency of TPA-heterobifunctional antibody complex increases as TPA concentration decreases. This observation may be considered in light of the relative affinities of TPA and antibody for fibrin as discussed above. Anti-fibrin antibody has a higher affinity for fibrin than does TPA. The heterobifunctional antibody is consequently capable of binding TPA to fibrin with an affinity greater than that which could be effected by TPA alone.

Thus a heterobifunctional antibody composed of a fibrin-specific antibody and an anti-TPA antibody bound to fibrin enhances the fibrinolytic potency of TPA, with increasing efficacy at decreasing TPA. This phenomenon is readily demonstrable in vitro at or below TPA plasma concentrations. Extending these observations in vivo, the treatment of thrombosis may be without the administration of exogenous plasminogen activators. Since the affinity of fibrin-specific antibodies for fibrin is greater than that of TPA, the risk of fibrinogenolysis or of the destruction of other clotting proteins is minimized and thereby the risk of bleeding is likely to be diminished.

## EXAMPLE 6

The following example shows the production of two different heterobifunctional antibodies from somatic cell fusion of two hybridoma lines. The resulting cell lines secrete asymetic antibodies (heterobifunctional antibodies) capable of binding both fibrin and TPA and sharing with the chemically produced product the ability to enhance fibrinolysis. Both heterobifunctional antibodies contained a TPA binding site and an additional binding site: one antibody possessed an antibody binding site specific for the amino terminus of fibrin's beta chain (F36.23); the other antibody possessed an antibody binding site specific for the amino terminus of fibrin's alpha chain (F32.1).

### ANTI-TPA, ANTI-BETA CHAIN HETEROBIFUNCTIONAL ANTIBODY

Hybridoma line TCL8 produces a monoclonal antibody specific for the catalytically active B chain of TPA. TCL8 cells were treated with 6-thioguanine to select for hypoxanthine phosphoribosyl transferase deficient (HPRT-MINUS) variants, subcloned and then tested for viability in HAT (hypoxanthine, aminopterin, thymidine) medium. Hybridoma line 59D8, which produces a monoclonal antibody specific for the beta chain of human fibrin, was grown in bromodeoxy uridine in order to select thymidine kinase deficient subclones that were not viable in HAT medium. The HPRT-MINUS and the thymidine kinase deficient subclones were fused in polyethylene glycol and desired clones selected that were viable in HAT medium. Further subclones were selected by screening with solid phase radioimmunoassay using a synthetic fibrin-like peptide and TPA as antigens. One cell line called F36.23 possessed both anti-human fibrin and anti-human TPA immunoreactivities. The products of this cell line were then purified to remove unproductive chain recombinants by serial affinity chromatography. Antibodies were first fractionated with beta chain peptide-Sepharose column and sequentially absorbed to and eluted from TPA-Sepharose. The purified products were then characterized by two additional assays that demonstrated their bifunctionality.

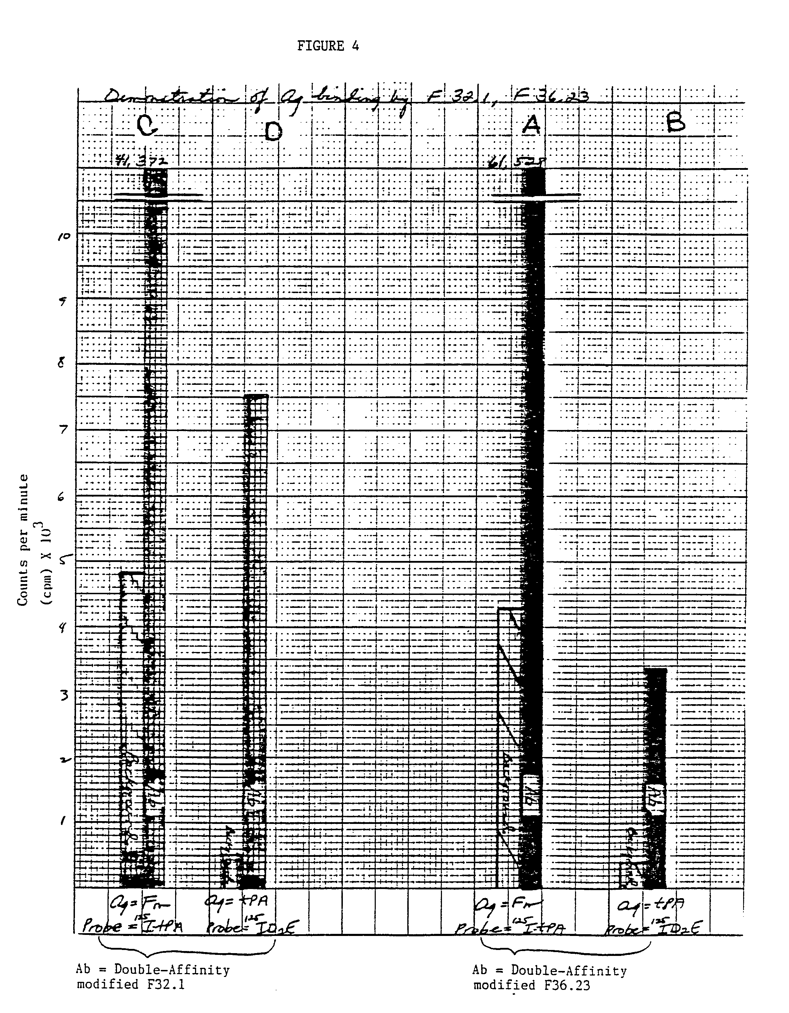

A solid-phase immunoradiometric assay was constructed in which fibrin monomer was adsorbed to a plastic surface, the test antibody solution added, followed by I25I labeled tPA (with intervening washing steps). Figure 4, column A shows evidence of binding to both fibrin and TPA. In Figure 4, column B, a similar assay was constructed in which TPA was adsorbed to the plate and I25I labeled D2E (a fragment of fibrin containing that amino terminus of the beta and alpha chains) was the probe. Here too the binding by the test antibody compared favorably to the control. These assays indicate that the purified product of clone F36.23 is a heterobifunctional antibody capable of binding both fibrin and TPA.

## ANTI-TPA, ANTI-ALPHA CHAIN HETEROBIFUNCTIONAL ANTIBODY

A second method was used to yield heterobifunctional antibody, F32.I. The F32.I cell line was selected after fusing TCL8 (HPRT-MINUS) cells with spleen cells from a mouse immunized with a fibrin-like peptide corresponding to the amino terminus of fibrin alpha-chains. Fusion products were similarly screened using TPA and an alpha-chain peptide as antigens to yield the desired hybridoma which produced both activities. The isotype of this monoclonal antibody was Gammal, Kappa.

After affinity chromatography similar to that described for antibody F36.23, except that an amino terminal alpha chain peptide was used instead of the beta chain peptide, a similar immunoradiometric assay was performed. Figure 4, column C, shows the binding of F32.I when fibrin monomer is bound to the solid phase and I25I tPA is the probe whereas Figure 4, column D, shows the binding of the same antibody when TPA is bound to the solid phase and I25I D2E is the probe. These assays also indicate that the purified product of clone F32.I is also a heterobifunctional antibody capable of binding both fibrin and TPA.

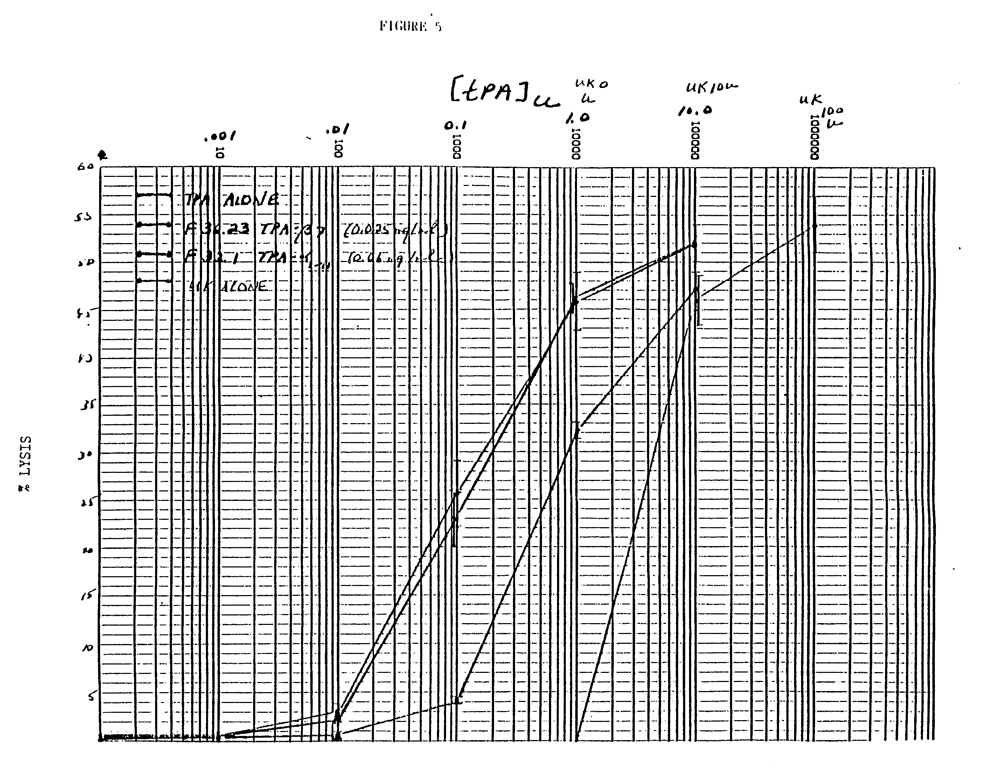

Both antibodies were tested for their ability to enhance fibrinolysis in an assay previously described above and in Bode et al., Science, 229:765-767 (1985). As is apparent from Figure 5, the potency of TPA in fibrinolysis is enhanced five- to ten-fold by the presence of either F36.23 or F32.I.

## Claims

I. A heterobifunctional antibody having dual specificity wherein one of the dual specificities is against a thrombus and the other specificity is against a thrombolytic agent.

2. The heterobifunctional antibody of claim I wherein the specificity against a thrombus is fibrin-specific substantially devoid of fibrinogen cross-reactivity.

3. The heterobifunctional antibody of claim I wherein the thrombolytic agent is selected from the group consisting of tissue-type plasminogen activator (TPA), streptokinase, and urokinase.

4. The heterobifunctional antibody of claim I wherein a thrombolytic agent is bound to said heterobifunctional antibody.

5. The heterobifunctional antibody of claim 4 wherein said thrombolytic agent bound to said antibody is selected from the group consisting of tissue-type plasminogen activator, streptokinase, and urokinase.

6. The heterobifunctional antibody of claim 4 wherein said thrombolytic agent bound to said antibody is radiolabelled or imaging labelled.

7. A heterobifunctional antibody having dual specificity comprising a modified antibody specific against a thrombus and a modified antibody specific against a thrombolytic agent.

8. The heterobifunctional antibody of claim 7 wherein the modified antibody having specificity against a thrombus is a fibrin-specific monoclonal antibody substantially devoid of fibrin cross-reactivity.

9. The heterobifunctional antibody of claim 7 wherein said modified antibody specific against a thrombolytic agent is an anti-TPA monoclonal antibody.

10. The heterobifunctional antibody of claim 7 wherein said modified antibody specific against a thrombolytic agent is an anti-streptokinase monoclonal antibody.

11. The heterobifunctional antibody of claim 7 wherein said modified antibody specific against a thrombolytic agent is an anti-urokinase monoclonal antibody.

12. A pharmaceutical composition comprising the heterobifunctional antibody of any one of claims 1 to 11 and a pharmaceutically acceptable carrier.

13. A heterobifunctional antibody according to any one of claims 1 to 11 for use in lysing a thrombus.

14. A heterobifunctional antibody according to any one of claims 1 to 11 for use in detecting a thrombus.

FIGURE 1

FIGURE 2

FIGURE 3

FIGURE 4

Demonstration of Ag binding by F 32.1, F 36.23

**Counts per minute (cpm) X 10³**

| C | D | A | B |

Ab = Double-Affinity
modified F32.1

Ab = Double-Affinity
modified F36.23

FIGURE 5